Europäisches Patentamt

European Patent Office

Office européen des brevets

(19)

(11) Veröffentlichungsnummer: **0 241 925 B1**

(12) **EUROPÄISCHE PATENTSCHRIFT**

(45) Veröffentlichungstag der Patentschrift: **16.09.92**

(51) Int. Cl.⁵: **A61F 13/66**, A61F 5/44

(21) Anmeldenummer: **87105568.7**

(22) Anmeldetag: **15.04.87**

(54) **Absorbierende Wegwerfunterhosen.**

(30) Priorität: **15.04.86 JP 87590/86**
**15.04.86 JP 87591/86**

(43) Veröffentlichungstag der Anmeldung:
**21.10.87 Patentblatt 87/43**

(45) Bekanntmachung des Hinweises auf die
Patenterteilung:
**16.09.92 Patentblatt 92/38**

(84) Benannte Vertragsstaaten:
**DE ES FR IT SE**

(56) Entgegenhaltungen:
**EP-A- 0 187 728**
**US-A- 4 205 679**

(73) Patentinhaber: **UNI-CHARM CORPORATION**
**182, Shimobun Kinsei-cho**
**Kawanoe-shi Ehime-ken(JP)**

(72) Erfinder: **Igaue, Takamitsu**
**385-1-3, Handa-otsu Kaneda-cho**
**Kawanoe-shi Ehime-ken(JP)**
Erfinder: **Inoue, Kohji**
**3496-2, Ohaza-Ueno Doi-cho**
**Uma-gun Ehime-ken(JP)**

(74) Vertreter: **Sperling, Rüdiger, Dipl.-Ing. et al**
**Patentanwälte Dipl.Ing.S. Staeger**
**Dipl.Ing.Dipl.Wirtsch.Ing. R Sperling Müller-**
**strasse 31**
**W-8000 München 5(DE)**

**Beschreibung**

Die Erfindung betrifft eine absorbierende Wegwerfunterhose mit einem Hauptkörper, bestehend aus einer wasserdurchlässigen, mit der Haut des Trägers in Kontakt kommenden Oberlage, einer der Hautseite der Oberlage abgewandten wasserundurchlässigen rückseitigen Lage und einem zwischen diesen beiden Lagen eingeschlossenen absorbierenden Kern und mit einander entsprechenden, in den seitlichen, sich im Schrittbereich gegenüberliegenden Abschnitten (gesehen in Draufsicht auf die Abwicklung des Hauptkörpers) ausgeschnittenen Beinöffnungen, mit einem Hüftbund und mit den Beinöffnungen zugeordneten elastischen Teilen, die die Ausschnittsbereiche umringen, wobei der Hauptkörper um eine den Körper in zwei vertikal benachbarte Hälften teilende Mittellinie X-X gefaltet und eine Verschweißung der seitlichen Ränder jeweils entlang einer durch eine Vertiefung definierten Linie ausgeführt ist.

In der EP 0187 728 ist eine gattungsgemäße Ausführungsform beschrieben, in der eine Vielzahl von parallel zueinander verlaufenden Schweißnähten vorgesehen sind, wobei die äußere der mehreren Schweißnähte unmittelbar neben der äußeren Randkante verläuft. Es ist qualitativ kein Abstand zwischen der ersten Schweißnaht und der freien Randkante gegeben, d.h. die am weitesten außen liegende Schweißnaht verläuft quasi unmittelbar auf der freien Randkante. Nachteilig bei dieser Ausführungsform ist, daß die Schweißnähte, die infolge des Aufschmelzens des Materials mit nachfolgenden Erstarren härter als das umliegende Material werden, ungeschützt der Berührung ausgesetzt sind, so daß beispielsweise die zarte Haut am Arm eines Babies von dem aufgeschmolzenen harten Material aufgekratzt werden könnte, jedenfalls aber unangenehme Berührempfindungen auslöst.

Bei einem weiteren Ausführungsbeispiel sind die jeweiligen Ränder mit ihren Außenlagen aufeinandergelegt und mittels einer Naht so miteinander verbunden, daß die freien Nahtkanten nach innen gerichtet sind.

Aus der US PS 4 205 679 ist eine Wegwerfunterhose bekannt, die vom Aufbau her als ein Stück ausgebildet ist und deren vorder- und rückseitige Abschnitte an den Längsrändern oder im seitlichen Bauchbereich miteinander verbunden sind. Diese Verbindung geschieht im wesentlichen durch eine gegenseitige sandwichartige Überlappung der Randbereiche, jeweils von entgegengesetzten Richtungen kommend, wobei der Detailaufbau im einzelnen variiert wird, so z.B. greifen die verschiedenen Lagen verzahnend ineinander, werden bei einem anderen Ausführungsbeispiel stumpf aneinander gestoßen oder die Randabschnitte der rückwärtigen Lagen umschließen die zugeordneten Randabschnitte der vorderseitigen Lagen oder umgekehrt.

Diese Ausführungsform hat den Nachteil, daß die jeweiligen Ränder in besonderer Weise in Position gebracht werden müssen, um eine entsprechende Verbindung zu ermöglichen.

Weiter sind aus der US-PS 4 427 408 absorbierende Unterhosen bekannt, die elastische Mittel im Hüftband und im Beinöffnungspaar aufweisen. Die bekannte Unterhose weist eine seitlich langgestreckte rechteckige wasserdurchlässige Oberlage, die so dimensioniert ist, daß sie den Vorder- und den rückwärtigen Bereich bilden kann, eine wasserundurchlässige rückwärtige Lage, die in Form und Abmessung identisch ist mit der Oberlage, und einen absorbierenden Kern auf, der zwischen den beiden Lagen eingeschlossen ist, wobei der Zentralbereich in der Mitte zwischen den seitlich einander gegenüberliegenden Enden der Lagen als Freiraum belassen ist, um den absorbierenden Kern mit den vorderen und rückwärtigen Bereichen zu verbinden. Des weiteren sind elastische Teile vorhanden, die entlang des oberen Endes der rückwärtigen Lage angeklebt sind, um die elastischen Teile mit einem Hüftband zu verbinden; das elastische Teil ist in ähnlicher Weise auf der rückwrätigen Lage entlang des anderen Endes befestigt, um mit den jeweiligen Beinöffnungen verbunden zu werden.

Der auf diese Weise hergestellte Hauptkörper wird entlang der Mittellinie dieses Freiraums zurückgefaltet und anschließend der vordere und rückwärtige Bereich entlang der Seitenränder, die dem Freiraum gegenüberliegen, miteinander verbunden, wobei zum Schluß der vordere und rückwärtige Bereich entlang des unteren Endes des Zentralabschnitts, der zwischen den beiden Seitenrändern und dem anderen, durch den zurückgefalteten Freiraum gebildeten Seitenrand definiert ist, miteinander verbunden werden, um auf diese Weise die vollständig zusammengesetzte Unterhose fertigzustellen.

Solche bekannten Unterhosen sind sicherlich vorteilhaft dadurch, daß die Herstellung vereinfacht ist, bergen aber verschiedene Nachteile in sich, beispielsweise daß die Funktion des Absorbierens unzulänglich ist, da die Komponenten im vorderen und rückwärtigen Bereich, die miteinander zu verbinden sind, im Schrittbereich konzentriert sind. Wenn diese Komponenten ohne Verbindung oder gebrochen sind, kommt es zu einem Verlust der gewünschten Funktion als Unterhose und zu einem Undichtwerden, da der Schrittbereich während der Bewegung der Beine des Trägers höchsten Zugkräften ausgesetzt ist. Diese Komponenten geben dem Träger das Gefühl eines unbequemen Paßsitzes.

Bei Windelhosen, in denen die Hüftbandabschnitte, die jeweils den vorderen und rückwärtigen Bereichen zugeordnet sind, in dem ringförmigen Band eingeschlossen sind, ist es unmöglich, eine überlappende Breite der seitlichen, einander gegenüberliegenden Ränder der vorderen und rückwärtigen Bereiche und eine passende Enge der benachbarten Bereiche einschließlich des Hüftbandes einzustellen, was demgegenüber bei Wegwerfwindeln des offenen Typs möglich ist, bei denen die Hüftbandabschnitte durch Bandbefestiger erst bei der tatsächlichen Verwendung der Windel miteinander verbunden werden. Als weiterer Nachteil ist anzumerken, daß in dem Bereich neben dem unteren Rand des Hüftbands in dem vorderen Bereich Falten gebildet werden, die infolge des lockeren Sitzes auftreten, wenn die Unterhosen getragen werden, da die Komponenten der Unterhosen steifer sind als diejenigen der Wegwerfwindel und weil die Bäuche der Babies allgemein nach vorne vorstehen.

Der Erfindung liegt die Aufgabe zugrunde, eine Wegwerfunterhose der genannten Art zu schaffen, die bei einfachem und kostengünstigen Aufbau bei hohem Tragekomfort ein besonders gutes Anliegen um den Körper gewährleistet.

Die Aufgabe wird erfindungsgemäß durch die im kennzeichnenden Teil des Hauptanspruchs angegebenen Merkmale gelöst.

Da die Linien, entlang denen die vorderen und rückwärtigen Bereiche miteinander verschweiß sind, von Nuten gebildet werden und die Ränder, die sich äußerhalb dieser Linien erstrecken, frei bleiben, kommen Abschnitte, die als Ergebnis des Schweißvorgangs gehärtet sind, nicht unmittelbar in Kontakt mit der Haut des Trägers, wodurch wirksam vermieden wird, daß diese Abschnitte dem Träger Schmerzen bereiten oder gar ihm Verletzungen zufügen.

Eine weiterer Vorteil der erfindungsgemäß aufgebauten Unterhosen liegt darin, daß die Ausschnittbereiche, die die jeweiligen Beinöffnungen bilden, in seitlich einander gegenüberliegenden Seitenbereichen des Schrittbereichs ausgebildet sind (bei Betrachtung einer Draufsicht auf die Abwicklung des Hauptkörpers) und daß die Beinöffnungen in die zugeordneten elastischen Teile eingearbeitet sind, so daß die jeweiligen Beinöffnungen von den zugeordneten elastischen Teilen umgeben sind, wenn die Unterhose vollständig zusammengesetzt ist. Diese Maßnahmen stellen sicher, daß die Beinöffnungen perfekt um das Bein herum passen und jegliche Leckage von Ausscheidungen verhindern.

In einem bevorzugten Ausführungsbeispiel der vorliegenden Erfindung erstrecken sich die zugeordneten elastischen Teile mit einer vorbestimmten Länge über die einander gegenüberliegenden Seitenränder des Kerns hinaus, der steifer ist als die obere Lage und die rückseitige Lage und zwar wegen seines komponenten Materials und seiner Dicke. Als Ergebnis dieser Ausbildung können die Verlängerungen der elastischen Teile die ihnen innewohnende Elastizität im wesentlichen frei von entgegengesetzten Einflüssen des starren Kerns entwickeln, wodurch der Abdichteffekt der Beinöffnungen um die Schenkel des Trägers verbessert wird und Leckage von Ausscheidungen in diesem Bereich wirksam verhindert werden.

Weitere vorteilhafte Ausgestaltungen der vorliegenden Erfindung sind, daß der rückwärtige Bereich größer dimensioniert ist als der vordere Bereich, daß die die Beinöffnungen definierenden Ausschnittsbereiche bezüglich der Vertikalmittellinie in Richtung des vorderen Bereichs versetzt sind und daß in einander gegenüberliegenden Seitenrändern (gesehen auf eine Abwicklung der Hose), Falze ausgebildet sind, die als Saum dienen, die ihrerseits an ihren Seitenränder miteinander verbunden sind; diese Merkmale dienen der Lösung der Probleme, die im Zusammenhang mit den bekannten Unterhosen aufgezeigt worden sind, wie beispielsweise das Problem, daß der Bereich entsprechend der Hüfte des Trägers einem Zug ausgesetzt ist, während der Bereich, der der Magengegend entspricht, wegen der Starrheit der Komponenten und der Figur des Trägers locker wird. Durch diese vorteilhaften Ausbildungen wird die Paßform weiter verbessert. Die seitliche Breite der diesbezüglichen unteren Abschnitte der vorderen und rückseitigen Bereiche sind größer dimensioniert als die Breite der entsprechenden Taillenbandabschnitte und der rückwärtige Bereich selber ist in Anbetracht des Merkmals, daß die Ausschnittsbereiche, die die jeweiligen Beinöffnungen in den seitlich einander gegenüberliegenden Bereichen des Schrittbereichs in Richtung des vorderen Bereichs der Unterhose versetzt sind, größer dimensioniert als dieser vordere Bereich.

Auf diese Weise können die Unterhosen während des Tragens einen guten Sitz um den Körper herum beibehalten, ohne die Nachteile oder Unannehmlichkeiten, daß der den Unterbauch betreffende Bereich zu eng wird, wegen der Lose, wodurch in dem Bereich unterhalb des Hüftbandes der Vorderbereich Falten wirft und die Unterhose als Ganzes herunterrutscht, selbst dann, wenn der Bauch nach vorne steht, wie es in der Regel bei Babies der Fall ist und der Hauptkörper der Unterhosen aus relativ starren Komponenten besteht.

Schließlich sind die jeweils den Taillenbandabschnitten und den Beinöffnungen zugeordneten elastischen Teile im wesentlichen parallel zueinander angeordnet und daher leicht in die zugeordneten Komponenten oder Unterhosen einzubauen, wodurch optimale Gegebenheiten zur Massenproduktion solcher Artikel bei geringen Kosten gegeben sind.

Im folgenden wird die Erfindung anhand in der Zeichnung dargestellter Ausführungsbeispiele näher erläutert. Es zeigen:

Fig. 1 eine perspektivische Ansicht eines ersten Ausführungsbeispiels einer erfindungsgemäßen Unterhosen,

Fig. 2 eine Vorderansicht dieser Unterhose nach dem Zusammensetzen,

Fig. 3 eine Draufsicht auf eine Abwicklung der Unterhose aus Fig. 2,

Fig. 4 eine Vorderansicht einer zweiten Ausführungsform einer zusammengesetzten erfindungsgemäßen Unterhose,

Fig. 5 eine Ansicht auf eine Abwicklung der Unterhose nach Fig. 4, und

Fig. 6 eine vergrößere Darstellung eines Schnitts entlang der Linie VI-VI in den Figuren 2 und 4.

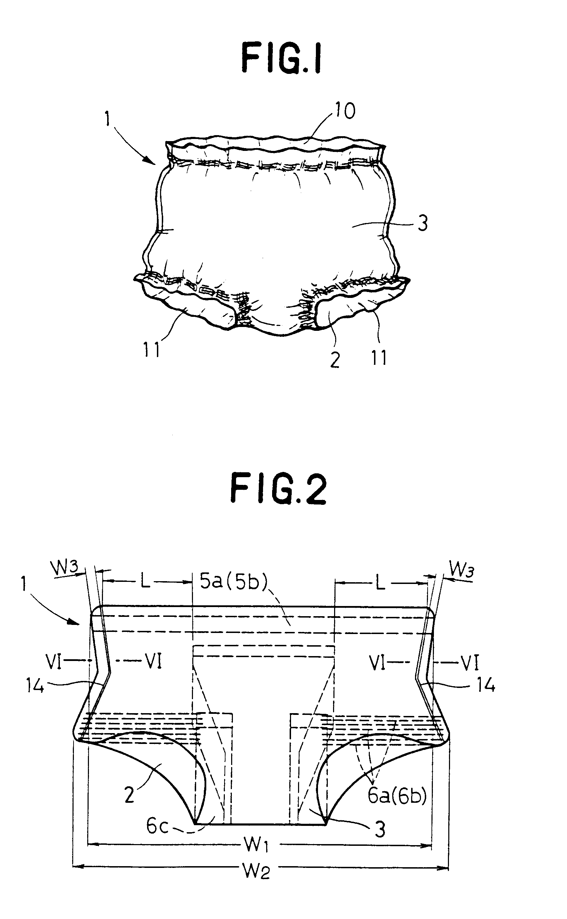

Es wird zunächst auf die Figuren 1 bis 3 Bezug genommen. Eine Wegwerfunterhose mit absorbierenden Eigenschaften weist einen Hauptkörper 1 mit einer wasserdurchlässigen Oberlage 2, eine hinsichtlich der Abmessungen identisch dazu ausgebildete wasserundurchlässige rückseitige Lage 3 und einen schmäler als diese beiden Lagen ausgebildeten absorbierenden Kern 4 auf. Der Hauptkörper 1 besitzt des weiteren elastische Teile 5a, 5b, die elastische Falten in zugeordneten Taillenbandabschnitten ausbilden, wobei die Taillenbandabschnitte gemeinsam ein Taillenband 10 bilden. Weitere elastische Teile 6a, 6b und 6c bilden elastische Kräuselfalten in Beinöffnungen 11.

Die Oberlage 2 besteht aus einem Faservlies mit zum Teil thermisch schweißbaren Fasern, während die rückseitige Lage 3 aus einem Kunststofffilm oder einem Laminat aus einem solchen Film und einem Faservlies besteht. Der Kern 4 weist eine flockige Pulpe oder eine Matte bestehend aus einer Mischung einer solchen Pulpe und hochabsorbierenden polymeren Partikeln auf, die an der Ober- und Unterseite der wasserundurchlässigen Lage abgedeckt ist (nicht dargestellt). Die elastischen Teile 5a, 5b und 5c sind aus einem Urethanschaum oder einem Kunststofffilm hergestellt, der unter Wärmebehandlung eine elastische Dehnbarkeit zeigt, die relativ weit sind, zum Beispiel 10 bis 45 mm, während die elastischen Teile 6a und 6b aus einer Vielzahl von Gummifäden bestehen. Die elastischen Teile 5a und 5b sind entlang senkrecht einander gegenüberliegender Enden der Oberlage 2, wie auch der rückseitigen Lage 3 in einem vorderen Bereich 7 und einen rückwärtigen Bereich 3 der Unterhosen entlang zugeordneter Taillenlinien 10a und 10b sandwichartig eingeschlossen, und zwar bevor die Unterhosen zusammengesetzt oder zurückgefaltet wird. Die elastischen Teile 5a, 5b 6a und 6b, insbesondere aber die elastischen Teile 6a und 6b erstrecken sich vorzugsweise nach außen über die jeweiligen Außenränder des Kerns 4 im vorderen Bereich 7 und im rückwärtigen Bereich 8 um 10 bis 130 mm hinaus, vorzugsweise um 30 bis 110 mm. Das am meisten bevorzugte Maß beträgt zwischen 50 und 95 mm.

Diese Länge ist mit dem Buchstaben L gekennzeichnet, die der Distanz von der entsprechenden Seitenkante des Kerns 4 bis zum inneren Seitenrand von Vertiefungen 14, entlang welchen das Verschweißen durchgeführt wird, entspricht. Diese Vertiefungen 14 werden nachfolgend näher erläutert.

In einem Schrittbereich sind die Oberlage 2 und die rückseitige Lage 3 an seitlich einander gegenüberliegenden Bereichen mit halb elliptisch geformten ausgeschnittenen Bereichen 12 versehen, die bezüglich einer Mittellinie X des Hauptkörpers der Unterhose in Richtung des vorderen Bereichs 7 versetzt sind und die entsprechenden Beinöffnungen 11 bilden. Daher ist in dem Hauptkörper 1 der rückwärtige Bereich 8 größer dimensioniert als der vordere Bereich 7. In den vorderen und rückwärtigen Bereichen 7 und 8 sind neben vertikal einander gegenüberliegender Enden der entsprechenden ausgeschnittenen Bereiche 12 die elastischen Teile 6a und 6b eingesetzt, die sich im wesentlichen parallel zu den elastischen Teilen 5a und 5b erstrecken. Neben seitlich einander gegenüberliegender Seitenränder der beiden herausgeschnittenen Bereiche 12 sind die vorderen und rückwärtigen Bereiche mit elastischen Teilen 6c versehen, die sich im wesentlichen quer zur Horizontalen erstrecken und die mit den zugeordneten elastischen Teilen 6a und 6b verbunden sind.

Diese elastischen Teile 6a, 6b und 6c umgeben auf diese Weise die zugeordneten Ausschnittsbereiche 12. Selbstverständlich ist es auch möglich, ein einziges elastisches Teil in einer gekrümmten Linie entlang der Kontur der zugeordneten Ausschnittsbereiche 12 herum zu befestigen. Das dargestellte Ausführungsbeispiel weist jedoch gegenüber einem solchen elastischen Einzelteil Vorteile auf, da die Operation des Anklebens der jeweiligen elastischen Teile im Herstellprozeß drastisch vereinfacht ist und auf diese Weise eine hohe Produktionsgeschwindigkeit erzielt werden kann.

Die vorderen und rückwärtigen Bereiche 7 und 8 sind entlang ihrer seitlich einander gegenüberliegenden Seitenränder mit Einkerbungen 13 in Form eines weit geöffneten V versehen, die als Saum oder Faltengeber den Sitz verbessern. Der Kern 4 ist zwischen der Oberlage 2 und der rückseitigen Lage 3 in relativ stationärer Lage festgeklebt, so daß er eine zentrale Zone der Lage mit einer schmalen Breite im Schrittbereich 9 und einer größeren Erstreckung im rückwärtigen Bereich 8 aufweist, welche Erstreckung größer ist als im vorderen Bereich 7.

Das Befestigen der Oberlage 2 und der rückseitigen Lage 3 entlang ihrer dazu bestimmten Seitenabschnitte,das Befestigen der elastischen Teile 5a, 5b, 6b, 6c auf die Oberlage 2 und/oder die rückseitige Lage 3 und das Befestigen des Kerns 4 auf die Oberlage 2 und/oder die rückseitige Lage 3 wird durch warmschmelzenden Klebstoff oder durch Warmverschweißen durchgeführt.

Die seitliche Breite $W_2$ des vorderen Bereichs 7 und des rückwärtigen Bereichs 8 entlang der unteren Enden ist an beiden Bereichen größer als die seitliche Breite $W_1$ entlang der Taillenbandabschnitte 10a und 10b.

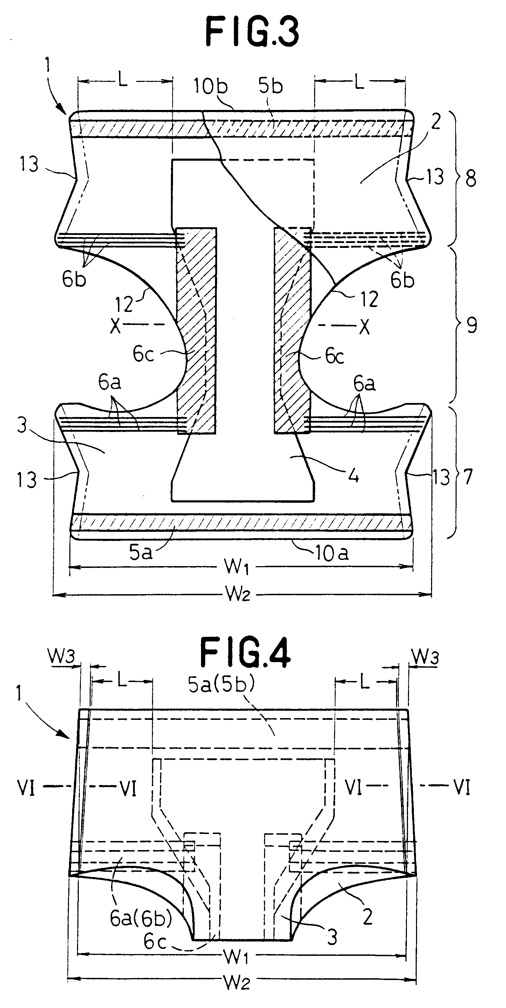

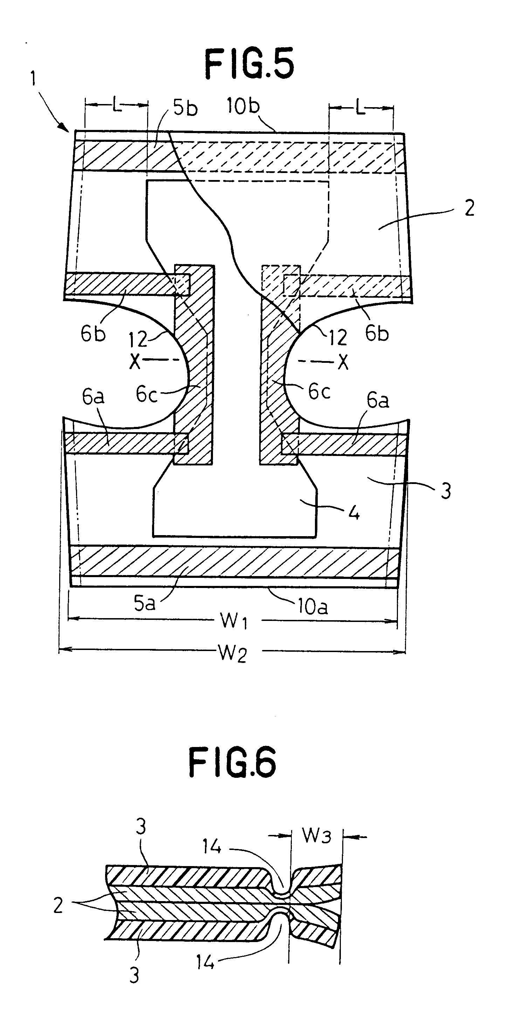

Es wird nun Bezug genommen auf die Figuren 4 und 5, in denen ein weiteres Ausführungsbeispiel des Hauptkörpers 1 dargestellt ist. Dieser Hauptkörper 1 entspricht im wesentlichen der Gestalt und dem Aufbau, wie er in Verbindung mit dem Ausführungsbeispiel gemäß der Figuren 1 bis 3 beschrieben worden ist, mit der Ausnahme, daß sowohl der vordere Bereich 7 als auch der rückwärtige Bereich 8 vom Schrittbereich 9 bis zum jeweiligen Taillenbandabschnitt 10a, 10b etwas kürzer sind. Die elastischen Teile 6a und 6b bestehen aus einem relativ breiten Urethanschaum oder Kunststoffilm, der nach einer Warmbehandlung elastische Eigenschaften besitzt.

Wie in den Figuren 1, 2 und 4 zu erkennen ist, wird der Hauptkörper 1 entlang der Mittellinie X (vergl. Fig. 3 und 5) in die vertikale Richtung zurückgefaltet, so daß sich die Oberlage 2 innen befindet. Anschließend wird entlang der sich gegenüberliegenden Seitenränder des vorderen Bereichs 7 und des rückwärtigen Bereichs 8 eine kontinuierliche Linie unter Druck verschweißt, wobei die äußersten Ränder mit einer geeigneten Breite W3, vorzugsweise etwas geringer als 10 mm breit, nicht verschweißt werden. In einem solchen Fall wird, um die Schweißbarkeit und somit die Schweißfestigkeit zu verbessern, vorzugsweise ein Kunststoffilm mit einem niederigeren Schmelzpunkt als derjenige der Oberlage 2 und der rückseitigen Lage 3 zwischen diesen beiden Lagen entlang der Schweißlinie oder zwischen den beiden Oberlagen, die nach dem Falten in Kontakt miteinander kommen, eingelegt.

**Patentansprüche**

1. Absorbierende Wegwerfunterhose mit einem Hauptkörper, bestehend aus einer wasserdurchlässigen, mit der Haut des Trägers in Kontakt kommenden Oberlage (2), einer der Hautseite der Oberlage abgewandten wasserundurchlässigen rückseitigen Lage (3) und einem zwischen diesen beiden Lagen eingeschlossenen absorbierenden Kern (4) und mit einander entsprechenden, in den seitlichen, sich im Schrittbereich gegenüberliegenden Abschnitten (gesehen in Draufsicht auf die Abwicklung des Hauptkörpers) ausgeschnittenen Beinöffnungen (11), mit elastischen Teilen (5a,5b) zum Ausbilden elastischer Falten jeweils in einem Hüftband (5) und mit den Beinöffnungen (11) zugeordneten elastischen Teilen, die die Ausschnittsbereiche umringen, wobei der Hauptkörper um eine den Körper in zwei vertikal benachbarte Hälften teilende Mittellinie X-X gefaltet und eine Verschweißung der seitlichen Ränder jeweils entlang einer durch eine Vertiefung (14) definierten Linie ausgeführt ist, dadurch **gekennzeichnet,**
   - daß die beiden Hälften entlang seitlich einander gegenüberliegender Ränder der Oberlageninnenseite verschweißt sind und
   - daß die den Beinöffnungen (11) zugeordneten elastischen Teile (6a,6b,6c) in Draufsicht auf den abgewickelten Hauptkörper aus mehreren geradlinig verlaufenden Teilabschnitten bestehen, nämlich ersten Teilabschnitten (6a;6b), die sich parallel zu einer den Hauptkörper in einen vorderen Teil und einen rückwärtigen Teil aufteilenden Mittellinie X-X und seitlich entlang einander gegenüberliegenden Enden der Ausschnittsbereiche (12) erstrecken und zweiten Teilabschnitten (6c), die sich senkrecht entlang seitlich einander gegenüberliegender Ränder der Ausschnittsbereiche erstrecken und mit den ersten Teilabschnitten (6a;6b) verbunden sind.

2. Unterhose nach Anspruch 1 oder 2, dadurch **gekennzeichnet,** daß sich die den Beinöffnungen (11) zugeordneten elastischen Teile, zumindest jedoch die ersten Teile, jeweils um 10 bis 130 mm über die Außenränder des Kerns (4) hinaus erstrecken.

3. Unterhose nach mindestens einem der Ansprüche 1 bis 3, dadurch **gekennzeichnet,** daß der Hauptkörper Einkerbungen (13) entlang der sich gegenüberliegenden Seitenränder und zwischen senkrecht einander gegenüberliegenden Enden in den entsprechenden Bereichen aufweist, die als Saum dienen.

4. Unterhose nach mindestens einem der Ansprüche 1 bis 4, dadurch **gekennzeichnet,** daß der Taille zugeordnete elastische Taillenbandteile (5a,5b) im wesentlichen parallel zu den ersten Teilabschnitten (6a,6b) der die Beinöffnungen (11) umgebenden elastischen Teile verlaufen.

5. Unterhose nach mindestens einem der Ansprüche 1 bis 5, dadurch **gekennzeichnet,** daß die jeweiligen zweiten Teile (6c) der elastischen Teile, die sich senkrecht entlang seitlich einander gegenüberliegender Ränder der jeweiligen Ausschnitte 12 für die Beinöffnung (11) erstrecken, im wesentlichen parallel zueinander verlaufen (gesehen in Draufsicht auf die Abwicklung des Hauptkörpers).

## Claims

1. Disposable absorbent underpants comprising a main body consisting of a water-permeable overlayer (2) which comes into contact with the skin, a water-impermeable reverse layer (3) spaced from the skin-side of the overlayer, and an absorbent core (4) enclosed within these two layers; corresponding cut-out leg openings (11) lying opposite each other in the crotch region when the opened out main body is seen in plan view; elastic parts (5a,5b) to form elastic folds in a waistband (5); and elastic parts corresponding to the leg openings (11) which surround the cut-out regions; whereby the main body is folded along a middle line X-X which divides the body into two vertical, adjacent halves, and the side edges are welded together along a line defined by a depression (14); **characterised** in that

- the two halves are welded together along facing side edges of the inner side of the overlayer;
- the elastic Parts (6a,6b,6c) corresponding to the leg openings (11) comprise several portions which are linear when the opened-out main body is seen in plan view, namely first portions (6a,6b) which extend parallel to a centre-line X-X which divides the main body into a forepart and a rear part and laterally along opposite ends of the cut-out regions (12), and second Portions (6c) which extend perpendicularly along opposite edges of the cut-out regions and are connected to the first portions (6a,6b).

2. Underpants as claimed in Claim 1, **characterised** in that the elastic parts corresponding to the leg openings (11), or at least the first portions, each extend from 10 to 130mm beyond the outer edges of the core (4).

3. Underpants as claimed in at least one of Claims 1 to 2, **characterised** in that the main body has notches (13) along the opposing side edges and between the perpendicularly opposing ends, in the corresponding positions which serve as seams.

4. Underpants as claimed in at least one of Claims 1 to 3, **characterised** in that the waistband parts (5a,5b) corresponding to the waist run essentially parallel to the first portions (6a,6b) of the elastic parts surrounding the leg openings (11).

5. Underpants as claimed in at least one of Claims 1 to 4, **characterised** in that the respective second portions (6c) of the elastic parts, which extend perpendicularly along opposite ends of the cut-outs for the respective leg openings (11), run essentially parallel to each other (when the opened out main body is seen in plan view).

## Revendications

1. Couche-culotte absorbante jetable, comprenant : un élément principal composé d'une couche supérieure (2) perméable à l'eau et venant en contact avec la peau du porteur, d'une couche arrière (3) imperméable à l'eau et orientée à l'opposé du côté de la couche supérieure tourné vers la peau, et d'un élément central (4) absorbant, prisonnier entre ces deux couches ; des ouvertures (11) de passage des jambes correspondant l'une à l'autre, découpées dans les parties latérales se faisant face (si l'on observe, par le dessus, le développement de l'élément principal) dans la zone de l'entre-jambes ; des éléments élastiques (5a, 5b) tendant à produire des fronces élastiques dans une zone de serrage des hanches (5) ; et d'autres éléments élastiques correspondant aux ouvertures (11) de passage des jambes, qui entourent les zones découpées, l'élément principal étant plié autour d'une ligne médiane X-X le partageant en deux moitiés verticales contiguës, et un soudage des bords latéraux étant exécuté le long d'une ligne définie par une dépression (14), couche-culotte caractérisée :

- en ce que les deux moitiés sont soudées le long de bords en opposition latérale mutuelle de la face intérieure de la couche supérieure, et ;
- en ce que les éléments élastiques (6a, 6b, 6c) correspondant aux ouvertures (11) de passage des jambes se composent, si l'on regarde, par le dessus, l'élément principal développé, d'une pluralité de sections rectilignes, à savoir : - des premières sections (6a, 6b) qui s'étendent parallèlement à une ligne médiane X-X partageant l'élément principal en une partie avant et une partie arrière, et latéralement le Long d'extrémités mutuellement opposées des zones découpées (12), - et des secondes sections (6c) qui s'étendent à la verticale le long de bords en opposition latérale mutuelle des zones découpées et qui sont jointes auxdites premières sections (6a, 6b).

2. Couche-culotte suivant la revendication 1, caractérisée en ce que les éléments élastiques correspondant aux ouvertures (11) de passage des jambes, et au moins les premières sections, se prolongent respectivement de 10 à 130 mm au-delà des bords périphériques de l'élément central (4).

3. Couche-culotte suivant au moins l'une des revendications 1 ou 2, caractérisée en ce que l'élément principal présente des échancrures (13) s'étendant le long des bords latéraux opposés et entre des extrémités verticalement opposées, dans les zones correspondantes qui servent d'ourlet.

4. Couche-culotte suivant au moins l'une des revendications 1 à 3, caractérisée en ce que les éléments élastiques de serrage de la taille (5a, 5b) prévus à la taille s'étendent substantiellement parallèlement aux premières sections (6a, 6b) des éléments élastiques qui entourent les ouvertures (11) de passage des jambes.

5. Couche-culotte suivant au moins l'une des revendications 1 à 5, caractérisée en ce que les secondes sections (6c) des éléments élastiques, qui s'étendent à la verticale des bords en relation mutuelle d'opposition latérales des découpures (12) prévues pour les ouvertures (11) de passage des jambes, sont substantiellement parallèles l'une à l'autre (si l'on observe, par le dessus, le développement de l'élément principal).

# FIG.1

# FIG.2

# FIG.3

# FIG.4

# FIG.5

# FIG.6